# EUROPÄISCHE PATENTSCHRIFT

(11) **EP 0 695 734 B1**
(45) Veröffentlichungstag und Bekanntmachung des Hinweises auf die Patenterteilung: **23.12.1998**
(21) Anmeldenummer: 95111805.8
(22) Anmeldetag: 27.07.1995
(51) Int. Cl.: C07C 45/50, C07C 45/80

(54) **Verfahren zur Herstellung von Aldehyden**
Process for the preparation of aldehydes
Procédé pour la préparation d'aldéhydes

(30) Priorität: 03.08.1994 DE 4427428
(43) Veröffentlichungstag der Anmeldung: 07.02.1996
(73) Patentinhaber: BASF AKTIENGESELLSCHAFT, 67056 Ludwigshafen (DE)
(72) Erfinder: Kneuper, Heinz-Josef, Dr., D-68163 Mannheim (DE); Paciello, Rocco, Dr., D-67098 Bad Dürkheim (DE); Röper, Michael, Prof. Dr., D-67157 Wachenheim (DE)

(56) Entgegenhaltungen:
- EP-A- 0 147 824
- EP-A- 0 156 253
- EP-A- 0 246 475
- EP-A- 0 588 225
- C.W. Kohlpainter: Dissertation, Technische Universität München, 1992, Seiten 123-139 (1992)
- JOURNAL ORGANOMET. CHEM., vol.193, no.C43, 1980
- JOURNAL ORGANOMET. CHEM., vol.192, no.C49, 1980
- ORGANOMETALLICS, vol.3, no., 1984, page 932
- ANGEW. CHEM., vol.105, no., 1993, page 1588
- ACS SYMP. SER., vol.486, no., 1992, page 229

## Beschreibung

Die vorliegende Erfindung betrifft ein Verfahren zur Herstellung von Aldehyden oder Aldehyden und Alkoholen durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe.

Die Hydroformylierung von Olefinen mit Kohlenmonoxid und Wasserstoff in Gegenwart von Übergangsmetall-Katalysatoren ist bekannt. Während α-Olefine sehr gut mit rhodiumhaltigen, phosphanmodifizierten Katalysatoren hydroformylierbar sind (vgl. J. Falbe, Ed.: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, S. 95 ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

Hingegen lassen sich außer α-Olefinen auch interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit phosphorhaltigen Liganden, wie Phosphinen oder Phosphiten, modifiziert sind. Solche nicht mit Phosphanen oder Phosphiten modifizierte Rhodium-Katalysatoren und deren Eignung als Katalysator zur Hydroformylierung der zuvor genannten Klassen von Olefinen sind bekannt (siehe Falbe, S.38 ff). Die Begriffe "nacktes Rhodium" oder "nackte Rhodium-Katalysatoren" werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphan- oder Phosphit-Liganden, modifiziert sind. Als Liganden in diesem Sinne werden nicht Carbonyl- oder Hydrido-Liganden verstanden. Es wird in der Fachliteratur (s. Falbe, S. 38 ff) angenommen, daß die Rhodium-Verbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungsreaktionszone nebeneinander ablaufenden Chemismen nicht eindeutig bewiesen ist. Lediglich der Einfachheit halber machen wir auch in dieser Anmeldung von dieser Annahme Gebrauch, ohne daß dadurch eine Einschränkung des Schutzumfangs der vorliegenden Anmeldung in Betracht zu ziehen wäre, falls sich zukünftig einmal eine andere Rhodiumspezies als die genannte, als die eigentlich katalytisch aktive herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonyl-Verbindungen, wie Rh₄(CO)₁₂ und Rh₆(CO)₁₆ oder aus Organo-Rhodium-Verbindungen, wie Rhodiumdicarbonylacetonylacetonat, Cyclooctadien-Rhodium-acetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen: US-A 4 400 547; DE-A 33 38 340; DE A-26 04 545; WO 82/03856; Chem. Ber. 102, 2238 (1969); Tetrahedron Lett. 29, 3261 (1968); Hydrocarbon Process. 54, Juni-Heft 85-86 (1975).

Allerdings hat auch die Hydroformylierung mit "nacktem" Rhodium den Nachteil, daß sich der thermolabile Rhodium-Katalysator (vgl. Us-A 4 400 547) infolge der thermischen Belastung bei der destillativen Aufarbeitung des Hydroformylierungsproduktes teilweise zu metallischem Rhodium zersetzt, das sich an den Wandungen des Reaktors und der Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

In DE-A 33 38 340 und US-A 4 400 547 werden Verfahren zur Hydroformylierung mittels "nackter Rhodium-Katalysatoren" beschrieben, bei denen zur Verhinderung der Rhodiumabscheidung dem Reaktionsaustrag der Hydroformylierung ein Phosphin oder Phosphit zugesetzt wird, welche den Rhodium-Katalysator durch die Bildung von Phosphan- und/oder Phosphit-Komplexen vor einer thermischen Zersetzung im Zuge der destillativen Aufarbeitung des Hydroformylierungsaustrags schützen. Nach Abschluß der Destillation wird der rhodiumhaltige Destillationssumpf mit einem Oxidationsmittel behandelt, wobei das Rhodium in katalytisch aktiver Form aus den betreffenden Phosphan- oder Phosphit-Komplexen freigesetzt wird und die Phosphan- und Phosphit-Liganden zu den entsprechenden, unter Hydroformylierungsbedingungen keine Rhodium-Komplexe bildenden Phosphanoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden.

WO 82/03856 betrifft ein Verfahren zur Thermostabilisierung von unmodifizierten, also "nackten Rhodium-Katalysatoren", in dem der Austrag aus der Hydroformylierungsreaktion mit einem sauerstoffhaltigen Gas behandelt wird, wodurch die gebildeten Aldehyde zum Teil zu den entsprechenden Carbonsäuren oxidiert werden, welche mit dem Rhodium-Katalysator bei der destillativen Aufarbeitung thermostabile Rhodium-Carboxylate bilden, die wieder als Katalysatoren zur Hydroformylierung verwendet werden können. Nachteilig an diesem Verfahren ist die Verringerung der Ausbeute, als Folge der partiellen Oxidation der Produktaldehyde zu carbonsäuren. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Zur Vermeidung von Rhodiumverlusten wurde gemäß US-A 3 984 478 ein Hydroformylierungsverfahren entwickelt, bei dem die Hydroformylierung in Gegenwart eines gegebenenfalls sulfonierten Phthalocyanins durchgeführt wird. Da die hierbei gebildeten Rhodium-Phthalocyanin-Komplexe z.T. schwer löslich oder nur in Wasser, nicht jedoch im organischen Hydroformylierungsmedium löslich sind, wird die Hydroformylierung wahlweise in Gegenwart der festen Rhodium-Phthalocyanine oder im Zwei-Phasen-System mit Wasser durchgeführt. Allerdings ist die koordinative Bindung des Rhodiums zum Phthalocyanin in diesen Komplexen sehr fest, so daß das Rhodium auch unter den Hydroformylierungsbedingungen ans Phthalocyanin gebunden bleibt. Als Folge hiervon findet die Hydroformylierungsreaktion nur an der Grenzfläche Hydroformylierungsmedium/ festes Phthalocyanin bzw. an der Grenzfläche zur wäßrigen Rhodium-Phthalocyanin-Komplexlösung statt, wodurch die Reaktionsgeschwindigkeit und damit auch die Raum-Zeit-Ausbeute der Hydroformylierungsreaktion so niedrig wird, daß sich dieses Verfahren nicht wirtschaftlich betreiben läßt.

DE-A 34 11 034 und DE-A 33 47 406 beschreiben die Abtrennung von Rhodiumcarbonylen und Rhodiumhydridocarbonylen, also "nacktem" Rhodium, mit Hilfe wasserlöslicher sulfonierter oder carboxylierter Phosphanliganden. Dabei wird das Rhodium mit Hilfe dieser Liganden von der organischen in die wäßrige Phase extrahiert und auf diese Weise vom in der organischen Phase löslichen Hydroformylierungsprodukt abgetrennt. Das auf diese Weise extrahierte, an die Phosphanliganden gebundene Rhodium muß, um wieder als "nacktes" Rhodium eingesetzt werden zu können, aus dem Komplex mit diesem Phosphanliganden herausgelöst werden, indem man es z.B. in das Salz einer höheren Carbonsäure überführt. Dieses Verfahren hat den Nachteil, daß es zusätzliche Stufen und Einsatzstoffe (Carbonsäuren) benötigt, um das komplexgebundene Rhodium in die betreffenden Rhodiumcarboxylate überzuführen. Als Alternative wird in diesen Schriften vorgeschlagen, die aus dem Lösungsmittel Wasser, Rhodium und Komplexbildner bestehende Phase unmittelbar als Katalysatorsystem für die Hydroformylierung zu verwenden. Doch auch diese Verfahrensweise birgt erhebliche Nachteile: So muß das Rhodium aus diesen Komplexen unter Hydrofomylierungsbedingungen herausgelöst und in "nacktes" Rhodium überführt werden. Abgesehen davon, daß durch die den komplexierten Rhodium-Katalysator enthaltende wäßrige Phase ein erheblicher Teil des Hydroformylierungsreaktorraums belegt wird, der dadurch für die Hydroformylierung nicht mehr nutzbar ist, wodurch ein größeres Reaktorvolumen benötigt wird, was wiederum zu höheren Anlagenkosten führt, findet im Zuge der Hydroformylierung unter den anzuwendenden Hydroformylierungsbedingungen ein Abbau der Phosphanliganden statt (vgl. Jamerson et al., J. Organomet. Chem. 193, C43 (1980); Jamerson et al., J. Organomet. Chem. 192, C49 (1980); Abatjoglou et al., Organometallics 3, 932 (1984); Abatjoglou et al., ACS Symp. Ser. 486, 229 (1992); W.A. Hermann et al: Angew. Chem. 105, 1588 (1993), insbesondere S. 1600ff). Da die Abbauprodukte der Phosphanliganden teilweise als hochwirksame Katalysatorgifte wirken (vgl. C.W. Kohlpaintner: Dissertation, Technische Universität München 1992, S. 123-139; insbesondere S. 137), müssen aufwendige Regenerierungsschritte unternommen werden, um das Rhodium und den Phosphanliganden von diesen Abbauprodukten abzutrennen, beispielsweise solche wie sie in DE-A 32 35 029 und DE-A 41 35 050 vorgeschlagen werden.

DE-A 34 12 335 betrifft ein Verfahren zur Hydroformylierung mit Hilfe von Rhodium-Katalysatoren, in denen das Rhodium an einen wasserlöslichen sulfonierten Triphenylphosphanliganden komplex gebunden ist. Unter den angegebenen Reaktionsbedingungen liegt kein "nacktes" Rhodium vor. Dementsprechend werden gemäß Beispielen nur 1-Olefine, jedoch keine internen Olefine hydroformyliert. Da langkettige Olefine in der wäßrigen, den komplexierten Rhodium-Katalysator enthaltenden Phase praktisch unlöslich sind, muß der Reaktionsmischung zusätzlich ein Lösungsmittler zugesetzt werden, da ansonsten die Produktivität des Verfahrens unbefriedigend ist. Anstelle eines Lösungsmittels wird in EP-A 173 219 zur Lösung dieses Problems die Anwendung von Ultraschall und in EP-A 226 988 die Anwendung einer sehr hohen Konzentration (25-30 Gew.-%) des sulfonierten Arylphosphan-Liganden in der wäßrigen Phase vorgeschlagen.

Zur direkten Herstellung interner, d.h. verzweigtkettiger Aldehyde, sogenannten Isoaldehyden, durch die Hydroformylierung von α-Olefinen, also Olefinen mit endständiger Doppelbindung, gibt es bislang kein wirtschaftlich zufriedenstellendes Verfahren. Bott (Fette, Seifen, Anstrichmittel 76, 443 (1974)) beschreibt die Synthese von internen Aldehyden aus α-Olefinen durch deren Isomerisierung mittels Kobaltoctacarbonyl (Co₂(CO)₈) unter einer Kohlenmonoxid-Atmosphäre bei 190°C oder an einem Natrium auf Aluminiumoxid-Katalysator und die nachfolgende Hydroformylierung der internen Olefine mittels Rhodium-Triphenylphosphin-Homogenkatalysatoren. Nachteilig ist hierbei die Verwendung zweier verschiedener Katalysatoren für die einzelnen Reaktionsschritte. Zudem reagieren interne Olefine mit dem Rhodium-Triphenylphosphan-Katalysator für technische Zwecke zu langsam.

Nach Fell et al (Tetrahedron Lett. 29, 3261 (1968)) wird Octen-1 zu internen Olefinen an einem aus Rh₂O₃ unter 200 bar (CO/H₂ - 1/1) Kaltdruck und bei 150°C in Hexan hergestellten Katalysator, welcher durch mehrmaliges Aufpressen von Kohlenmonoxid von Wasserstoff befreit worden ist, bei 100 bar Kohlenmonoxid-Druck und 190°C isomerisiert. Nachteilig an diesem Verfahren ist die aufwendige Katalysatorherstellung und die Verwendung eines inerten Lösungsmittels, welches einen erheblichen Teil des zur Verfüguna stehenden Hochdruck-Reaktionsraumes in Anspruch nimmt und daher die Raum-Zeit-Ausbeute erniedrigt. Weiterhin wurde von Eine Studie über kinetische und mechanistische Untersuchungen zur Isomerisierung von α-Olefinen zu internen Olefinen und deren Hydroformylierung ist in Kogyo Kagaku Zasshi, 72, 671 (1969) veröffentlicht.

EP-A 588 225 lehrt ein Verfahren zur Hydroformylierung mit "nackten" Rhodiumkatalysatoren, bei dem das "nackte" Rhodium aus dem Hydroformylierungsaustrag in eine wäßrige Lösung stickstoffhaltiger Komplexbildner extrahiert wird. Die das komplexierte Rhodium enthaltende wäßrige Extraktionslösung wird sodann in die Hydroformylierungsstufe zurückgeführt, wo unter den Hydroformylierungsbedingungen "nacktes" Rhodium aus den Rhodiumkomplexen freigesetzt wird und in die organische Phase des Hydroformylierungsmediums migriert.

Der vorliegenden Erfindung lag daher die Aufgabe zugrunde, ein Verfahren zur Herstellung von Aldehyden aus langkettigen und/oder verzweigten Olefinen mit Hilfe "nackter Rhodium-Katalysatoren" zu finden, mit dem zum einen die Probleme der Abscheidung metallischen Rhodiums bei der destillativen Aufarbeitung des Hydroformylierungsproduktes sowie der Rhodium-Katalysator-Abtrennung von nicht destillierbaren Produktaldehyden zufriedenstellend gelöst werden können. Es sollte zu diesem Zweck nach einem Hydroformylierungsverfahren gesucht werden, in dem sich Komplexliganden reversibel und in Abhängigkeit vom Druck der Kohlenmonoxid-Wasserstoff-Gasmischung an den "nackten" Rhodium-Katalysator koordinativ binden, so daß dieser bei einer extraktiven Aufarbeitung stabilisiert und extrahierbar wird. Nach der Extraktion sollte der komplexierte Rhodium-Katalysator wieder in "nacktes" Rhodium umgewandelt werden. Dabei war besonderes Augenmerk darauf zu legen, daß keine Schädigung des Komplexliganden stattfindet. Weiterhin sollten Rhodium-Verluste im Zuge der Extraktion minimiert und ein kombiniertes Hydroformylierungs-Extraktionsverfahren gefunden werden, das die Hydroformylierung interner Olefine wirtschaftlich und mit hohen Raum-Zeit-Ausbeuten gestattet, ohne mit den Nachteilen der vorgenannten Verfahren behaftet sein.

Eine weitere Aufgabe war es, ein Verfahren zu finden, das die Herstellung von Isoaldehyden aus α-Olefinen auf wirtschaftliche Weise gestattet. Dabei sollten insbesondere die zuvor geschilderten Nachteile des Standes der Technik vermieden werden.

Dementsprechend wurde ein Verfahren zur Herstellung von Aldehyden oder Aldehyden und Alkoholen durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe, gefunden, das dadurch gekennzeichnet ist, daß man den Rhodium-Katalysator aus dem Hydroformylierungsaustrag mittels einer wäßrigen Lösung eines wasserlöslichen, phosphorhaltigen Komplexbildners ausgewählt aus der Gruppe der einfach oder mehrfach sulfonierten Mono- und Oligophosphane, der einfach oder mehrfach sulfonierten Oligophosphite und/oder aus der Gruppe der einfach oder mehrfach carboxylierten Mono- oder Oligophosphane, in die wäßrige Phase extrahiert und aus dem extrahierten Hydroformylierungsaustrag den Aldehyd oder den Aldehyd und den Alkohol isoliert, den wäßrigen rhodiumhaltigen Extrakt einer Vorcarbonylierungsstufe zuführt und ihn in der Vorcarbonylierungsstufe in Gegenwart von einer im wesentlichen wasserunlöslichen, organischen Flüssigkeit und in Gegenwart von Kohlenmonoxid oder eines Kohlenmonoxid-haltigen Gasgemischs bei einem Druck von 5 bis 600 bar und bei einer Temperatur von 50 bis 180°C einer Vorcarbonylierung unterzieht, den Austrag aus der Vorcarbonylierungsstufe in eine den Hauptteil des Rhodiums enthaltende organische Phase und in eine den Komplexbildner enthaltende wäßrige Phase trennt und die organische Phase zur Hydroformylierung des Olefins in Gegenwart von Synthesegas bei 50 bis 1000 bar und bei einer Temperatur von 60 bis 180°C der Hydroformylierungsstufe zuführt.

Erfindungsgemäß werden die bei der Hydroformylierung mit "nacktem Rhodium" erhaltenen rhodiumhaltigen Hydroformylierungsausträge mit wasserlöslichen, phosphorhaltigen Komplexbildnern, beispielsweise ein oder mehrzähnigen Komplexbildnern, versetzt, die mit dem Rhodium-Katalysator Komplexe bilden, welche hydrophil sind und infolge ihrer guten Wasserlöslichkeit mit Wasser den Rhodium-Katalysator aus dem organischem Medium des Hydroformylierungsaustrags extrahieren können. Nach der extraktiven Abtrennung des im Hydroformylierungsaustrag enthaltenen Rhodium-Katalysators in Form eines wasserlöslichen Komplexes mit dem erfindungsgemäß eingesetzten Komplexbildner, kann das Hydroformylierungsprodukt auf an sich übliche Weise aufgearbeitet werden, beispielsweise indem man das Hydroformylierungsprodukt aus dem organischen Extrakt destillativ isoliert oder indem man leichter flüchtige organische Bestandteile des Hydroformylierungsaustrags vom schwerer flüchtigen oder gegebenenfalls sogar undestillierbaren Hydroformylierungsprodukt abdestilliert. Der wäßrige Extrakt des Hydroformylierungsaustrags, welcher das nunmehr vom phosphorhaltigen Komplexbildner komplexierte Rhodium enthält, wird einer Verarbeitungsstufe zugeleitet, in der das komplexierte Rhodium in Gegenwart einer im wesentlichen wasserunlöslichen, organischen Flüssigkeit und in Gegenwart von Kohlenmonoxid oder eines Kohlenmonoxid-haltigen Gasgemisches bei einem Druck von im allgemeinen 5 bis 600 bar, vorzugsweise von 10 bis 400 bar und besonders bevorzugt von 20 bis 300 bar und bei einer Temperatur von 50 bis 150°c, vorzugsweise von 70 bis 130°C, insbesondere von 80 bis 110°C, carbonyliert wird. Durch die Carbonylierung wird das Rhodium aus dem hydrophilen Komplex mit dem phosphorhaltigen Komplexbildner gelöst, und die sich dabei bildende, lipophile Rhodiumcarbonylverbindung migriert in die wasserunlösliche, organische Flüssigkeit. Dieser Schritt wird als "Vorcarbonylierung" bezeichnet, da die Carbonylierung des Rhodiums nicht im Hydroformylierungsreaktor selbst, sondern eben in der diesem vorgeschalteten Vorcarbonylierungsstufe erfolgt. Statt dem vorstehend angegebenen Druck kann in der Vorcarbonylierungsstufe auch ein höherer Druck angewandt werden, ohne daß dies die Durchführbarkeit der Vorcarbonylierung beeinträchtigt, beispielsweise kann bei einem Druck von 5 bis 1000 bar gearbeitet werden, besonders vorteilhaft ist jedoch das Arbeiten bei den zuvor angegebenen Druckbereichen.

Der Austrag aus der Vorcarbonylierungsstufe kann leicht, z.B. in einem Phasenabscheider, in eine den Hauptteil des Rhodiums in Form einer Rhodiumcarbonylverbindung enthaltende organische Phase und in eine den Komplexbildner enthaltende wäßrige Phase getrennt werden. Die organische Phase wird anschließend der Hydroformylierungsstufe zugeführt, in der das in der organischen Phase enthaltene Rhodium in den "nackten" Rhodium-Katalysator überführt wird, der die Hydroformylierung des zu hydroformylierenden Olefins katalysiert. Die den Komplexbildner enthaltende, wäßrige Phase kann anderweitig verwendet werden, beispielsweise vorteilhaft zur Extraktion des Rhodium-Katalysators aus dem Hydroformylierungsaustrag.

Die Verwendung einer solchen Vorcarbonylierungsstufe führt zu erheblichen Vorteilen. Da die Carbonylierung im allgemeinen schneller und unter milderen Bedingungen als die Hydroformylierungsreaktion erfolgt, kann zum einen der phosphorhaltige Komplexbildner weniger stringenten Reaktionsbedingungen während einer kürzeren Verweilzeit ausgesetzt und auf diese Weise geschont werden, zum anderen kann bei gleicher Raum-Zeit-Ausbeute mit kleineren Reaktordimensionen gearbeitet werden, da die wäßrige Phase nicht mehr in den Hydroformylierungsreaktor gelangt. Der aufwendige Einsatz von Hilfsmitteln, wie Lösungsvermittler, Ultraschall oder hochkonzentrierter wäßriger Lösungen des Komplexliganden, ist im erfindungsgemäßen Verfahren ebenfalls nicht erforderlich.

Die Vorcarbonylierung kann mit Hilfe von Kohlenmonoxid, Synthesegas oder eines Kohlenmonoxid-haltigen Gasgemisches durchgeführt werden.

Als Kohlenmonoxid-haltige Gasgemische werden im Sinne dieser Anmeldung Kohlenmonoxid-haltige Gasgemische verstanden, die nicht unter den Begriff "Synthesegas" fallen, beispielsweise CO/H₂-Gemische mit von Synthesegas verschiedener Zusammensetzung oder Gemische des Kohlenmonoxids mit anderen, unter den Reaktionsbedingungen inerten Gasen, wie Stickstoff, Edelgasen oder niederen Kohlenwasserstoffen, wie Methan, Ethan, Propan oder Butan. Der Gehalt derartiger Kohlenmonoxid-haltiger Gasgemische an inerten Gasen ist im allgemeinen kleiner als 60 Vol.%, vorzugsweise kleiner als 50 Vol.%, insbesondere kleiner als 30 Vol.%. Bei Verwendung solcher Inertgas-haltiger Kohlenmonoxid-Gasgemisch kann es, in Abhängigkeit von der Zusammensetzung dieser Gasgemische, insbesondere bei einem hohen Inertgasgehalt, erforderlich sein, die Vorcarbonylierung bei einem höheren Druck und/oder bei einer höheren Temperatur als den für die Durchführung der Vorcarbonylierung als vorteilhaft bezeichneten Druck- und Temperaturwerten durchzuführen, damit eine zufriedenstellende Umsatzrate gewährleistet ist. Die optimalen Bedingungen können hierzu in einem einfachen Vorversuch ermittelt werden.

Vorzugsweise wird zur Vorcarbonylierung jedoch Kohlenmonoxid verwendet, das je nach Herkunft, technisch bedingt, weniger als 10 Vol%, vorzugsweise weniger als 5 Vol.%, insbesondere weniger als 1 Vol.% an Inertgasen oder Wasserstoff enthalten kann.

Als im wesentlichen wasserunlösliche, organische Flüssigkeit können erfindungsgemäß eine Vielzahl von unter den Reaktionsbedingungen der Vorcarbonylierungsstufe und der Hydroformylierungsstufe inerten Flüssigkeiten verwendet werden, wobei "inert" bedeutet, daß diese Flüssigkeiten nicht den Ablauf der Vorcarbonylierung oder der Hydroformylierung beeinträchtigen.

Als derartige organische Flüssigkeiten können beispielsweise Kohlenwasserstoffe verwendet werden. Vorzugsweise werden aber Aldehyde oder Alkohole oder Gemische aus Aldehyden und Alkoholen verwendet. Beispielsweise kann zu diesem Zweck ein Teil des rohen Austrags aus der Hydroformylierungsstufe verwendet werden, es können aber auch die in der Hydroformylierungsstufe gebildeten und nachfolgend isolierten Aldehyde oder Alkohole oder deren Gemische verwendet werden. Es besteht bezüglich der Art der als wasserunlösliche, organische Flüssigkeit in der Vorcarbonylierungsstufe verwendeten Aldehyde praktisch keine Beschränkung. Vorzugsweise werden aber solche Aldehyde oder Alkohole verwendet, wie sie bei der Hydroformylierung des zu hydroformylierenden Olefins entstehen.

Als im wesentlichen wasserunlösliche, organische Flüssigkeit können in der Vorcarbonylierungsstufe auch sogenannte Hochsieder eingesetzt werden. Dies sind hochsiedende Kondensationsprodukte von Aldehyden, die im Zuge der Hydroformylierung als Nebenprodukte entstehen. Es handelt sich hierbei naturgemaß im allgemeinen um Vielkomponentenmischungen. In US-A 4 148 830 wird die chemische Natur derartiger Hochsiedergemische beispielhaft erläutert. Derartige Hochsiedergemische sind auch im Handel erhältlich, beispielsweise unter der Bezeichnung Texanol^{®} von der Firma Eastman.

Besonders bevorzugt werden im erfindungsgemäßen Verfahren Olefine als im wesentlichen wasserunlösliche, organische Flüssigkeit in die Vorcarbonylierungsstufe eingesetzt. Obgleich bezüglich der Art des in der Vorcarbonylierung eingesetzten Olefins prinzipiell keine Beschränkung besteht, werden vorzugsweise solche Olefine eingesetzt, wie sie in der nachfolgenden Hydroformylierungsstufe eingesetzt werden.

Es kann sich auch als vorteilhaft erweisen, den gesamten Olefinzulauf für die Hydroformylierungsstufe zunächst durch die Vorcarbonylierungsstufe zu schicken. Bei der Verwendung von Kohlenmonoxid oder Kohlenmonoxid-haltigen Gasgemischen als Carbonylierungsagens können sich Acyl-Komplexe des carbonylierten Rhodiums mit dem Olefin bilden, wodurch eine zusätzliche Stabilisierung des homogen gelösten Rhodiums erzielt werden kann.

Es wurde überraschenderweise festgestellt, daß beim Einsatz von α-Olefinen in die Vorcarbonylierungsstufe, eine Isomerisierung der α-Olefine zu internen Olefinen stattfinden kann. Somit können α-Olefine erfindungsgemäß zu internen Olefinen isomerisiert und diese in der nachfolgenden Hydroformylierungsstufe zu internen, also verzweigten, Aldehyden hydroformyliert werden. Dies ist besonders vorteilhaft, da α-Olefine auf dem Markt in größeren Mengen zur Verfügung stehen als interne Olefine, α-Olefine zudem preiswerter erhältlich sind als interne Olefine und verzweigte Aldehyde und verzweigte Alkohole gesuchte Zwischenprodukte zur Herstellung von verzweigten Carbonsäuren, Alkoholen und Aminen sind, welche wiederum in großem Umfang z.B. als Zusätze für Wasch- und Reinigungsmittel und zur Herstellung von biologisch abbaubaren Tensiden verwendet werden.

Zur Herstellung von verzweigten Alkoholen und/oder Aldehyden aus α-Olefinen gemäß dem erfindungsgemäßen Verfahren wird zweckmäßigerweise der α-Olefinzulauf durch die Vorcarbonylierungsstufe geschickt. Die Vorcarbonylierung und simultan dazu die Isomerisierung des α-Olefins zum internen Olefin wird im allgemeinen innerhalb der für die Vorcarbonylierungsstufe angegebenen Temperatur- und Druckbereiche ausgeführt, wobei zweckmäßigerweise ein Druck von weniger als 100 bar und eine Temperatur von über 110°C eingestellt wird. Die zur vollständigen Isomerisierung des α-olefins erforderliche Verweilzeit des α-olefins in der Vorcarbonylierungsstufe ist im allgemeinen von den darin angewandten Reaktionsbedingungen abhängig und wird zweckmäßigerweise durch einen Vorversuch bestimmt.

Selbstverständlich können nach dem erfindungsgemäßen Verfahren auch α-Olefine zu n-Aldehyden hydroformyliert werden, beispielsweise indem man das α-Olefin unter Umgehung der Vorcarbonylierungsstufe in den Hydroformylierungsreaktor einleitet oder indem man die Vorcarbonylierung bei einem Druck von mehr als 100 bar und bei einer Temperatur von unter 110°C durchführt.

Die Vorcarbonylierungsstufe kann aus einem oder mehreren, parallel oder hintereinander geschalteten Reaktoren bestehen. Bei einer diskontinuierlichen Betriebsweise können hierzu herkömmliche Rührautoklaven und bei einer kontinuierlichen Betriebsweise Kaskaden-Rührautoklaven oder Rohrreaktoren, die zur Durchmischung des Reaktionsgutes geeigneten Vorrichtungen, wie Rührer oder statische Mischer, enthalten, verwendet werden.

Der Austrag aus der Vorcarbonylierungsstufe wird in einer geeigneten Vorrichtung, z.B. einem Phasenabscheider, in eine wäßrige und eine organische Phase getrennt. Die Phasenabscheidung kann unter erhöhtem Druck, beispielsweise unter dem Betriebsdruck der Vorcarbonylierungsstufe, oder bei Atmosphärendruck, nach vorausgegangener Entspannung des Austrags aus der Vorcarbonylierung erfolgen. Da sowohl die Vorcarbonylierung als auch die Hydroformylierung unter erhöhtem Druck erfolgen, wird die Phasentrennung vorteilhaft ebenfalls unter Druck vorgenommen.

Die so aus dem Austrag der Vorcarbonylierungsstufe abgetrennte organische Phase, die das zur Katalyse der Hydroformylierung benötigte Rhodium und je nach Art der in der Vorcarbonylierungsstufe verwendeten wasserunlöslichen, organischen Flüssigkeit das zu hydroformylierende Olefin oder eine andere geeignete organische Flüssigkeit enthält, wird der Hydroformylierungsstufe zugeführt. Wurde keine Entspannung und Entgasung des Vorcarbonylierungsaustrags vorgenommen, enthält die organische Phase weiterhin das in der Vorcarbonylierungsstufe verwendete gasförmige Carbonylierungsagens zum Teil in gelöster Form.

Die Hydroformylierung wird mit Hilfe des in der Vorcarbonylierungsstufe erzeugten, carbonylierten Rhodiums in Gegenwart von Synthesegas durchgeführt. Als Synthesegas werden gemeinhin Kohlenmonoxid/Wasserstoff-Gasgemische mit einem Wasserstoffgehalt von 10 bis 90 Vol.-%, insbesondere 40 bis 60 Vol.-%, verstanden. Erforderlichenfalls wird der Hydroformylierungsstufe noch das zu hydroformylierende Olefin zugeführt, falls dieses der Hydroformylierungsstufe nicht schon mit der organischen Phase aus dem Vorcarbonylierungsaustrag zugeführt wurde.

Die Hydroformylierung wird im allgemeinen bei Temperaturen von 60 bis 180°C, vorzugsweise 80 bis 150°C und besonders bevorzugt bei 90 bis 130°C und bei einem Druck von im allgemeinen 50 bis 1000 bar, vorzugsweise bei 70 bis 500 bar, insbesondere bei 100 bis 400 bar durchgeführt. Die Hydroformylierung erfolgt ansonsten unter Bedingungen, wie sie üblicherweise bei Hydroformylierungen mit "nacktem" Rhodium angewandt werden und wie sie beispielsweise in der eingangs zitierten Literatur, betreffend die Hydroformylierung mit "nacktem" Rhodium, beschrieben sind.

In Abhängigkeit von den in der Hydroformylierungsstufe angewandten Druck- und Temperaturbedingungen und der Synthesegaszusammensetzung kann das Produktverhältnis Alkohol/Aldehyd im Hydroformylierungsaustrag beeinflußt werden. Beispielsweise wird bei der Hydroformylierung von Trimerpropylen bei jeweils gleichen Synthesegaszusammensetzungen - CO/H₂-Molverhältnis 50/50, 40/60 bzw. 60/40 - bei 130°C und einem Druck von 280 bar ein Aldehyd/Alkohol-Molverhältnis von jeweils 93/7 erhalten. Bei Erhöhung der Temperatur von 130°C auf 150°C verändert sich das Aldehyd/Alkohol-Molverhältnis im Hydroformylierungsaustrag in Abhängigkeit von der Synthesegaszusammensetzung - CO/H₂-Molverhältnis 50/50, 40/60 bzw. 60/40- auf 76/24, 67/33 bzw. 82/18.

Die Hydroformylierung kann in An- oder Abwesenheit von organischen Lösungsmitteln durchgeführt werden. Besonders vorteilhaft ist die Verwendung organischer Lösungsmittel beispielsweise bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende aromatische und aliphatische Kohlenwasserstoffe oder auch hochsiedende Aldehyd-Kondensationsprodukte, die im Zuge der Hydroformylierungsreaktion als Nebenprodukt als Folge der Kondensation der Produktaldehyde entstehen.

Der Austrag aus der Hydroformylierungsstufe wird vor seiner Extraktion mit der wäßrigen Lösung des phosphorhaltigen Komplexbildners zweckmäßigerweise entspannt. Die Extraktion des Hydroformylierungsaustrags wird im allgemeinen bei Temperaturen von 80 bis 140°, vorzugsweise von 90 bis 130°C, insbesondere 100 bis 120°C und bei einem Druck von im allgemeinen 1 bis 20 bar, vorzugsweise 1 bis 10 bar und besonders bevorzugt von 1 bis 5 bar durchgeführt. Die Extraktion kann an der Luft oder unter einer Inertgasatmosphäre durchgeführt werden, beispielsweise einer Stickstoff-, Wasserstoff- oder Argonatmosphäre, es kann aber auch vorteilhaft sein, dem verwendeten Inertgas zusätzlich Kohlenmonoxid oder Synthesegas beizumischen oder die Extraktion in Gegenwart von Kohlenmonoxid durchzuführen.

Zur Extraktion des Rhodium-Katalysators aus dem Hydroformylierungsaustrag kann eine frische wäßrige Lösung des Komplexbildners verwendet werden, vorzugsweise wird hierfür aber die bei der Phasentrennung des Vorcarbonylierungsaustrag erhaltene, den gelösten Komplexbildner enthaltende wäßrige Phase verwendet, die zu diesem Zweck in die Extraktionsstufe zurückgeführt wird.

Bei der Extraktion wird im allgemeinen ein Volumenverhältnis wäßrige/organische Phase von im allgemeinen 0,2 bis 2, vorzugsweise von 0,3 bis 1, eingestellt. Das Molverhältnis phosphorhaltiger Komplexbildner/Rhodium beträgt bei der Extraktion im allgemeinen 5:1 bis 10000:1, vorzugsweise 10:1 bis 5000:1, insbesondere 50:1 bis 1000:1.

Zur Extraktion des Hydroformylierungsaustrags mit der wäßrigen Lösung des Komplexbildners sind praktisch alle Flüssig-flüssig-Extraktionsapparaturen geeignet, beispielsweise Mixer-Settler-Apparaturen, Blasensäulen oder Gegen- oder Gleichstromextraktionskolonnen, wobei diese noch mit zusätzlichen Einbauten zur besseren Durchmischung von wäßriger und organischer Phase versehen sein können, beispielsweise mit Siebböden, Füllkörpern oder statischen Mischern. Die Extraktion des Rhodium-Katalysators aus dem Hydroformylierungsaustrag kann einstufig durchgeführt werden, vorzugsweise wird eine Mehrstufenextraktion angewandt, beispielsweise eine Zwei- oder Dreistufenextraktion, wobei die wäßrige, den Komplexbildner enthaltende Phase im Gleichstrom oder, besonders bevorzugt, im Gegenstrom bezüglich der organischen Phase geführt werden kann.

Nach beendeter Extraktion kann der vom Rhodium-Katalysator befreite Hydroformylierungsaustrag auf an sich herkömmliche Weise, beispielsweise destillativ, zur Isolierung der darin enthaltenen Wertprodukte-Alkohole und oder Aldehyde- aufgearbeitet werden.

Eine vorteilhafte Ausgestaltung des erfindungsgemäßen Verfahrens wird in Fig. schematisch dargestellt und wird im folgenden erläutert. An sich selbstverständliche Anlagendetails, die zu der Veranschaulichung des erfindungsgemäßen Verfahren nicht erforderlich sind, wurden aus Gründen der Übersichtlichkeit nicht in Fig. aufgenommen. Die in Fig. dargestellte Ausgestaltung des erfindungsgemäßen Verfahrens umfaßt die Verfahrensstufen der Hydroformylierung, eine zweistufige Gegenstromextraktion des Hydroformylierungsaustrags mittels Mixer-Settler-Apparaturen und die Vorcarbonylierungsstufe. Es versteht sich von selbst, daß an Stelle der Mixer-Settler-Apparaturen auch andere der oben erwähnten Extraktionsapparaturen eingesetzt werden können.

In der Ausgestaltung des erfindungsgemäßen Verfahrens gemäß Fig. wird der Hydroformylierungsaustrag aus dem Hydroformylierungsreaktor 1 über Leitung 2 nach Entspannung und gegebenenfalls nach Zufuhr von Inertgas (nicht eingezeichnet) in die Extraktionsstufe A, umfassend die Mixer-Settler-Apparatur 3/4, eingetragen und dort mit der über Leitung 19 zugeführten wäßrigen Lösung des phosphorhaltigen Komplexbildners aus Extraktionsstufe C (Mixer-Settler-Apparatur 6/7) extrahiert. Beim Anfahren der Anlage oder zum Zwecke der Ergänzung der Komplexbildnerlösung kann frische Komplexbildnerlösung über einen nicht in Fig. eingezeichneten Einlaß z.B. dem Mixer 3 zugeführt werden. Das im Mixer 3 enthaltene Extraktionsgemisch wird im Settler 4 in eine erste organische und eine erste wäßrige Phase aufgetrennt. Die erste wäßrige Phase wird über Leitung 8 in den Vorcarbonylierungsreaktor geleitet, wohingegen die erste organische Phase über Leitung 5 der Extraktionsstufe C zugeführt wird. Vor Einleitung in den Vorcarbonylierungsreaktor 11 wird die erste wäßrige Phase noch in geeigneten Mischapparaturen über die Zuläufe 9 und 10 mit einer im wesentlichen wasserunlöslichen, organischen Flüssigkeit, beispielsweise rohem Hydroformylierungsaustrag, Texanol^{®} oder vorzugsweise dem zu hydroformylierenden Olefin, und dem Carbonylierungsagens, also Kohlenmonoxid oder einem geeigneten Kohlenmonoxid-haltigen Gasgemisch, vorzugsweise mit Kohlenmonoxid vermischt. Es ist grundsätzlich auch möglich die über die Leitungen 9 und 11 zugeführten Einsatzstoffe direkt in den Vorcarbonylierungsreaktor 10 einzuleiten. Im Vorcarbonylierungsreaktor 11 wird das an den phosphorhaltigen Komplexbildner gebundene, in der wäßrigen Phase befindliche Rhodium unter den angegebenen Bedingungen carbonyliert und das sich dabei bildende, lipophile Rhodiumcarbonylverbindung migriert in die organische Phase. Der Austrag aus dem Vorcarbonylierungsreaktor 11 wird über Leitung 12, vorzugsweise ohne vorherige Entspannung, in den Phasenabscheider 13 geleitet und dort in eine zweite organische und eine zweite wäßrige Phase getrennt (Phasenabscheidung B).

Die zweite organische Phase, die außer der wasserunlöslichen, organischen Flüssigkeit noch das zur Katalyse der Hydroformylierung benötigte Rhodium und gegebenenfalls überschüssiges Carbonylierungsagens gelöst enthält, wird über Leitung 14 in den Hydroformylierungsreaktor 1 geleitet. Über Leitung 15 wird dem Hydroformylierungsreaktor Synthesegas zugeleitet, welches alternativ auch direkt in Hydroformylierungsreaktor 1 geleitet werden kann. Wurde im Vorcarbonylierungsreaktor als wasserunlösliche, organische Flüssigkeit kein Olefin verwendet, so kann das zu hydroformylierende Olefin entweder direkt über Leitung 16 in den Hydroformylierungsreaktor 1 eingeleitet werden oder vorher über einen nicht in Fig. eingezeichneten Einlaß dem Strom in Leitung 14 zugemischt werden. Im Hydroformylierungsreaktor wird das Olefin unter den angegebenen Bedingungen zu den entsprechenden Alkoholen und/oder Aldehyden hydroformyliert.

Die zweite wäßrige Phase aus Phasenabscheidung B, die die an Rhodium abgereicherte Lösung des phosphorhaltigen Komplexbildners enthält, wird über Leitung 17, gegebenenfalls nach vorheriger Entspannung, dem Mixer 6 zugeführt. In Extraktionsstufe C, umfassend den Mixer 6 und den Settler 7, wird die erste organische Phase aus Extraktionsstufe A mit der zweiten wäßrigen Phase aus Phasenabscheidung B nochmals extrahiert, um Restmengen von Rhodium aus der ersten organischen Phase zu entfernen. Die im Mixer 6 enthaltene Extraktionsmischung wird im Settler 7 in eine dritte organische und eine dritte wäßrige Phase getrennt. Die nunmehr von Rhodium befreite dritte organische Phase wird über Leitung 18 zur weiteren Aufarbeitung zwecks Isolierung der Wertprodukte - Alkohol und/oder Aldehyd - ausgetragen. Die dritte wäßrige Phase aus Extraktion C wird über Leitung 19 in die Extraktionsstufe A geleitet, womit der Kreislauf geschlossen ist.

Die Erstbefüllung des Reaktors mit Rhodium kann durch Einführung einer Lösung oder Suspension des Rhodiumkatalysators oder eine der zur Herstellung des Rhodium-Katalysators geeigneten, eingangs erwähnten Vorläuferverbindungen, beispielsweise in den Vorcarbonylierungsreaktor 11 oder in den Hydroformylierungsreaktor 1, erfolgen. Gleiches gilt für gegebenenfalls erforderliche Ergänzungen von verbrauchtem Katalysator. Es ist auch möglich das Rhodium über Leitung 20 oder anderen, in Fig. nicht eingezeichneten Einlässen, beispielsweise über einen Einlaß an Leitung 8, in die Anlage einzuleiten.

Als Komplexbildner, die mit dem im Austrag der Hydroformylierungsreaktion gelösten Rhodium-Katalysator wasserlösliche Komplexe bilden, werden vorzugsweise sulfonierte, phosphorhaltige Komplexbildner, aus der Gruppe der einfach oder mehrfach sulfonierten Mono- und Oligophosphane oder der einfach oder mehrfach sulfonierten Mono- oder Oligophosphite, und/oder aus der Gruppe der einfach oder mehrfach carboxylierten Mono- oder Oligophosphane oder der einfach oder mehrfach carboxylierten Monooder Oligophosphite, ausgewählt.

Unter Einwirkung dieser Komplexbildner bilden sich vermutlich über das freie Elektronenpaar der Phosphoratome koordinative Bindungen mit dem Rhodium-Zentralatom des Rhodium-Katalysators aus, wobei vermutlich ein Teil des an das zentrale Rhodiumatom des Rhodium-Katalysators gebundenen Kohlenmonoxids unter den Bedingungen der Komplexbildung durch diese Liganden reversibel verdrängt wird. Die Anwesenheit von Sulfonat- oder Carboxylatgruppen in den erfindungsgemäß verwendeten, phosphorhaltigen Komplexbildnern ist kritisch für die Durchführbarkeit des erfindungsgemäßen Verfahrens. Die Komplexbildner können eine oder mehrere Carboxylat- und/oder Sulfonat-Gruppen pro Molekül enthalten, wobei die Anzahl der Carboxylat- und/oder Sulfonat-Gruppen im Molekül selbstverständlich auch von der Molekülgröße des Komplexbildners und dessen Reaktivität gegenüber Sulfonierungsreagenzien abhängig ist. Die Anzahl der Carboxyl- und/oder Sulfonsäure-Gruppen im Komplexbildner-Molekül beeinflußt die Wasserlöslichkeit dieser Komplexbildner. Selbstverständlich können im erfindungsgemäßen Verfahren auch phosphorhaltige Komplexbildner eingesetzt werden, die sowohl Carboxylgruppen als auch Sulfonsäuregruppen als Substituenten enthalten oder Gemische sulfonierter und carboxylgruppenhaltiger Komplexbildner verwendet werden. Die Sulfonatund Carboxylat-Gruppen liegen in den erfindungsgemäß angewandten Komplexbildnern vorzugsweise in Salzform vor, insbesondere in Form von wasserlöslichen Salzen, besonders bevorzugt in Form ihrer Onium-, Alkalimetall- und/oder Erdalkalimetallsalze. So können z.B. wasserlösliche Ammonium-, Phosphonium- oder Arsoniumsalze der betreffenden Carbon- oder Sulfonsäuren eingesetzt werden. Zur Vermeidung von Mißverständnissen sei an dieser Stelle ausdrücklich darauf hingewiesen, daß die erfindungsgemäß verwendbaren phosphorhaltigen Komplexbildner-Sulfonate oder -Carboxylate die Sulfonat- oder Carboxylatgruppen als Substituenten tragen und nicht etwa salzartig mit irgendwelchen Sulfonat- oder Carboxylatanionen verbunden sind.

Vorzugsweise werden im erfindungsgemäßen Verfahren sulfonierte Arylphosphane, insbesondere sulfonierte Triarylphosphane, sulfonierte Triaralkylphosphane oder alicyclische, sulfonierte Arylsubstituenten tragende Phosphane eingesetzt. Des weiteren ist der Einsatz sulfonierter Arylphosphite, insbesondere sulfonierter Triarylphosphite, bevorzugt. Diese Arylgruppen-haltigen Komplexbildner lassen sich besonders einfach, nach an sich gängigen Methoden, zum Beispiel durch die Sulfonierung der entsprechenden Phosphane oder Organophosphite mittels konzentrierter Schwefelsäure oder Schwefelsäure/Schwefeltrioxid-Mischungen, die auch als Oleum bezeichnet werden, oder mittels Chlorsulfonsäure herstellen.

Bei der Sulfonierung dieser Phosphane oder Organophosphite nach den zuvor angegebenen Methode können je nach Art des als Ausgangsmaterial verwendeten Phosphans oder Phosphits, Gemische aus einfach oder mehrfach sulfonierten Produkten entstehen. Diese aus verschiedenerlei sulfonierten Verbindungen bestehenden Sulfonierungsprodukte können selbstverständlich nach Verfahren des Standes der Technik, wie Kristallisation oder Ionenaustauschchromatographie, in die einzelnen, sulfonierten Komponenten aufgetrennt und die so erhaltenen Einzelkomponenten als Komplexbildner im erfindungsgemäßen Verfahren eingesetzt werden. Da aber die sulfonierten Einzelverbindungen im allgemeinen keine wesentlich besseren Eigenschaften als Komplexbildner haben als das Produktgemisch der Sulfonierung, wird zweckmäßigerweise das sulfonierte Produktgemisch als Komplexbildner verwendet, wodurch sich die Auftrennung des Gemisches in seine Einzelkomponenten erübrigt.

Beispielhaft für im erfindungsgemäßen Verfahren als Komplexbildner anwendbare wasserlösliche, sulfonierte Phosphane seien genannt: Diphenylphosphinphenyl-m-sulfonsäure, Phenylphosphindi-(m-phenylsulfonsäure), Triphenylphosphin-tri(m-sulfonsäure) (Herstellung s. DE-A 32 35 030; DE-A 34 31 643; Schindlbauer: Monatsh. Chem. 96, 2051 (1965), W.A. Hermann et al: Angew. Chem. 105, 1588 (1993), W.A. Hermann et al: J. Organomet. Chem. 339, 85 (1990), sulfonierte Tris(ω-phenylalkyl)phosphine (Herstellung: Bartik et al: Organometallics 12, 164 (1993)), sulfonierte 2,2'-Bis-(diphenylphosphanomethyl)-biphenyle, wie sie einschließlich ihrer Herstellung in EP-A 4 912 40 und bei W.A. Hermann et al: J. Mol. Cat. 73, 191 (1992) beschrieben sind, 3,4-Dimethyl-2,5,6-tris(m-sulfonatophenyl) -1-phosphanorbornadien (DE-A 42 20 267). Weitere als Komplexbildner geeignete sulfonierte, wasserlösliche Phosphane werden beispielsweise in W.A. Hermann et al., Angew. Chem. 105, 1588 (1993), Barton et al., J. Coord. Chem. 24, 43 (1991) und Kalck et al., Adv. Organomet. Chem. 34, 219 (1992) offenbart. Andere Herstellmethoden für wasserlösliche, sulfonierte Phosphane werden von Sinou, Bull. Soc. Chim. France 480 (1987) beschrieben. Die vorgenannten Zitate werden hinsichtlich der darin beschriebenen sulfonierten, wasserlöslichen Phosphane als Referenz in diese Anmeldung einbezogen. Die Arylgruppen dieser sulfonierten Phosphane können zusätzlich zur Sulfonsäuregruppe noch 1 oder 2 unter den Reaktionsbedingungen inerte Sübstituenten tragen, beispielsweise C₁- bis C₄-Alkylgruppen, C₁- bis C₄-Alkoxygruppen, Halogenatome, insbesondere Fluor-, Chlor- oder Bromatome, Hydroxygruppen, Nitrogruppen, Carboxylgruppen oder Nitrilgruppen. Vorzugsweise werden aber die unsubstituierten, sulfonierten Phosphane verwendet. Besonders vorteilhaft werden diese sulfonierten Phosphane in Form ihrer wasserlöslichen Salze mit Alkali- oder Erdalkalimetallen, vorzugsweise mit Alkalimetallen, insbesondere in Form ihrer Lithium-, Natrium- oder Kaliumsalze, verwendet. Sie können auch in Form ihrer wasserlöslichen Ammoniumsalze eingesetzt werden.

Als solche können beispielsweise carboxylierte Phosphane verwendet werden, die in ihrem Kohlenstoffgrundgerüst den obengenannten sulfonierten Triarylphosphanen entsprechen, anstelle der Sulfonsäuregruppen jedoch 1 bis 3 Carboxylgruppen tragen. Solche carboxylierten Triarylphosphane können z.B. nach den Verfahren von Gilman et al., J. Am. Chem. Soc. 67, 824 (1945), Luckenbach et al., Z. Naturforsch B, 32, 1038 (1977) oder Schumann et al., Synth. Comm. 22, 841 (1992) hergestellt werden.

Für das erfindungsgemäße Verfahren geeignete wasserlösliche, carboxylierte Phosphanliganden sind weiterhin Liganden der allgemeinen Formel

Ar₃₋ₙP (CH₂COOH)ₙ I

in der Ar für einen gegebenenfalls substituierten, vorzugsweise einen unsubstituierten oder einen mit einer Carboxylgruppe substituierten Phenylring steht und n eine ganze Zahl von 1 bis 3 bedeutet sowie Liganden der allgemeinen Formel II 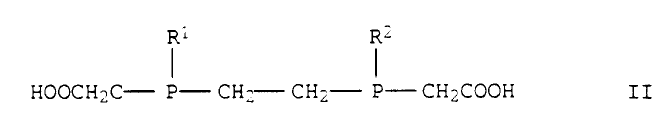 in denen R¹ und R² gleich oder verschieden sind und für einen unsubstituierten oder gegebenenfalls substituierten, vorzugsweise einen unsubstituierten oder einen mit einer Carboxylgruppe substituierten Phenylring, oder eine -CH₂-COOH-Gruppe stehen und Liganden der allgemeinen Formel III 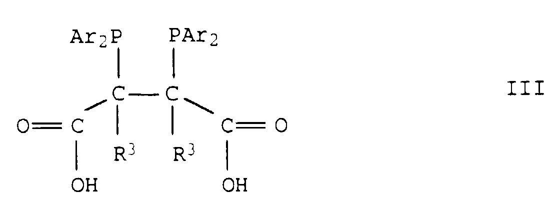 in der Ar für einen gegebenenfalls substituierten, vorzugsweise einen unsubstituierten oder einen mit einer Carboxylgruppe substituierten Phenylring steht und in der die Reste R³ gleich sind und Wasserstoff bedeuten oder gemeinsam miteinander eine chemische Bindung bilden.

Beispielhaft für Verbindungen der allgemeinen Formeln I, II und III seien Diphenylphosphinopropionsäure (Herstellung: Mann et al., J. Chem. Soc. 4453 (1957)), P,P'-Diphenylethylendiphosphino-P,P'-diessigsäure der Formel 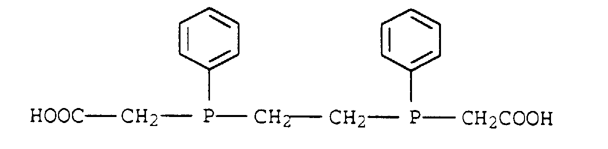 herstellbar nach Podlahava et al., J. Inorg. Nucl. Chem. 40, 967 (1978), Ethylendiphosphino-P,P,P',P'-tetraessigsäure der Formel 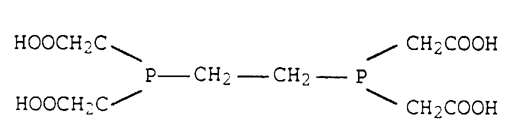 herstellbar nach Podlahava et al., Coll. Czech. Chem. Comm. 45, 2049 (1980),
2,3-Bis(diphenylphosphino)maleinsäure der Formel 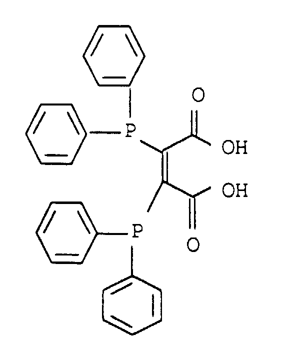 herstellbar nach Avey et al., Inorg. Chem. 32, 233 (1993) sowie die daraus durch Hydrierung der Doppelbindung erhältliche 2,3-Bis(diphenylphosphino)bernsteinsäure.

Die carboxylierten Phosphanliganden werden vorzugsweise in Form ihrer wasserlöslichen Salze, insbesondere in Form ihrer wasserlöslichen Alkalimetallsalze, vorzugsweise in Form ihrer Lithium-, Natrim-, oder Kaliumsalze, oder in Form ihrer wasserlöslichen Ammoniumsalze im erfindungsgemäßen Verfahren eingesetzt.

Anstelle sulfonierter oder carboxylierter wasserlöslicher Phosphane oder in Gemischen mit diesen können im erfindungsgemäßen Verfahren als Komplexbildner auch wasserlösliche, sulfonierte Organophosphite verwendet werden. Da diese Organophosphite in wäßrigem Medium eingesetzt werden, müssen sie eine hinreichende Hydrolysestabilität haben. Solche hydrolysestabile, sulfonierte Organophosphite und deren Herstellung werden beispielsweise in Fell et al., J. Prakt. Chem. 335, 75 (1993) beschrieben, worauf hiermit verwiesen wird. Anstelle der von Fell et al. beschriebenen liphophilen Ammoniumsalze der sulfonierten Phosphit-liganden werden im erfindungsgemäßen Verfahren deren wasserlösliche Alkalimetallsalze verwendet.

Besonders bevorzugt werden im erfindungsgemäßen Verfahren als Komlexbildner Triphenyl-tri(m-sulfonsäure) sowie die wasserlöslichen, sulfonierten Derivate des 2,2'-Bis-(diphenylphosphanomethyl)-biphenyl, sowie deren wasserlösliche Salze, insbesondere deren Alkalimetallsalze, vorzugsweise deren Lithium-, Natrium-, oder Kaliumsalze eingesetzt. Unter wasserlöslichen, sulfonierten Derivaten des 2,2'-Bis-(diphenylphosphanomethyl)-biphenyl IV 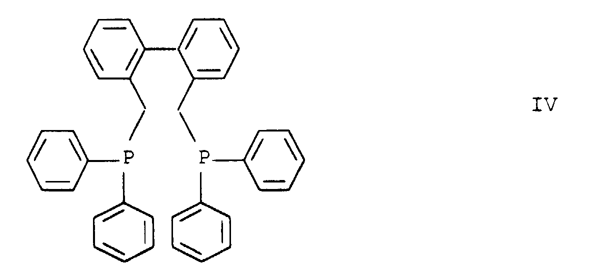 werden die bei der Sulfonierung von IV mittels Schwefelsäure/ Schwefeltrioxid-Mischungen erhaltenen Gemische der vierfach, fünffach und sechsfach sulfonierten Verbindungen IV verstanden (vgl. J. Mol. Cat. 73, 191 (1992)). Diese Gemische können ohne weitere Auftrennung in ihre unterschiedlich stark sulfonierten Einzelkomponenten als Komplexbildner im erfindungsgemäßen Verfahren eingesetzt werden. Wie erwähnt werden diese Gemische der sulfonierten Derivate von IV vorzugsweise in Form ihrer wasserlöslichen Alkalimetallsalze eingesetzt.

Wie aus den vorangegangenen Darlegungen hervorgeht, können eine Vielzahl von sulfonierten oder carboxylierten Phosphanen oder, soweit diese hydrolysestabil sind, von sulfonierten oder carboxylierten Organophosphiten als Komplexbildner im erfindungsgemäßen Verfahren verwendet werden. Es ist hierzu zu bemerken, daß es bei Verwendung dieser Komplexbildner lediglich auf deren Eigenschaft ankommt, mit dem in der organischen Phase des Hydroformylierungsaustrags gelösten "nackten" Rhodium wasserlösliche Komplexe zu bilden, die einerseits ausreichend stabil sind, um das extrahierte Rhodium in der wäßrigen Phase zu stabilisieren, andererseits unter den Bedingungen der Vorcarbonylierungsstufe, das komplexierte Rhodium wieder freigeben, so daß dieses nach Carbonylierung des Rhodiums wieder in die organische Phase migrieren kann. Irrelevant für ihre Verwendung als Komplexbildner im erfindungsgemäßen Verfahren sind die Eigenschaften mancher der genannten Komplexbildner die Hydroformylierungseigenschaften zu modifizieren, da im erfindungsgemäßen Verfahren "nacktes" Rhodium als Hydroformylierungskatalysator dient und nicht ein Komplex des Rhodiums mit einem der vorgenannten Liganden.

Das erfindungsgemäße Verfahren eignet sich besonders gut zur Hydroformylierung von Olefinen mit mehr als 3, vorzugsweise mehr als 7 Kohlenstoffatomen, insbesondere zur Hydroformylierung von C₇- bis C₂₀-Olefinen, die geradkettig oder verzweigt sein können und die α-olefinische und/oder interne Doppelbindungen enthalten können, z.B. Octen-1, Dodecen-1, Trimer- und Tetramerpropylen, oder Dimer-, Trimer- und Tetramerbutylen. Ebenso können ungesättigte Oligomere anderer Olefine hydroformyliert werden, desgleichen Cooligomere verschiedener Olefine. Die aus diesen Olefinen gebildeten Aldehyde dienen z.B. als Vorstufen für die Herstellung von Weichmacheralkoholen und Tensiden, die daraus auf an sich herkömmliche Weise durch Hydrierung erzeugt werden können. Die zur Hydroformylierung eingesetzten Olefine können z.B. durch die säurekatalysierte Wasserabspaltung aus den entsprechenden Fettalkoholen oder nach einer Vielzahl anderer technischer Verfahren, wie sie beispielsweise in Weissermel, Arpe: Industrielle Organische Chemie, S.67-86, Verlag Chemie, Weinheim, 1978, dargestellt sind, erhalten werden. Werden im erfindungsgemäßen Verfahren α-Olefine eingesetzt, so können diese wahlweise durch direkten Eintrag in die Hydroformylierungsstufe zu den entsprechenden n-Aldehyden hydroformyliert oder als Folge von deren Eintrag in die Vorcarbonylierungsstufe, nach ihrer Isomerisierung zu internen Olefinen, zu Isoaldehyden hydroformyliert werden, auf deren Anwendung bereits hingewiesen wurde.

Besonders gut geeignet ist das erfindungsgemäße Verfahren auch zur Hydroformylierung von polymeren Olefinen, beispielsweise niedermolekularem Polyisobuten, niedermolekularem Polybutadien oder niedermolekularem 1,3-Butadien-Isobuten- oder Buten-Copolymeren. Unter "niedermolekularen" Polymeren werden insbesondere Polymere mit Molgewichten von 500 bis 5000 Dalton verstanden. Es können aber auch höhermolekulare, ungesättigte Polymere hydroformyliert werden. Einzige Voraussetzung hierfür ist, daß diese im Hydroformylierungsmedium löslich sind. Die Hydroformylierungsprodukte dieser polymeren Olefine, insbesondere die des niedermolekularen Polyisobutens, können beispielsweise nach dem Verfahren der EP-A 244 616 durch reduktive Aminierung in die entsprechenden Amine umgewandelt werden, die als Kraftstoffadditiv Verwendung finden. Niedermolekulares Polyisobuten ist beispielsweise nach dem Verfahren von EP-A 145 235 erhältlich, niedermolekulare Isobuten-1,3-Butadien-Copolymere können z.B. nach dem Verfahren der deutschen Patentanmeldung DE-A 43 06 384 gewonnen werden.

Das erfindungsgemäße Verfahren eignet sich praktisch zur Herstellung aller Aldehyde, die auf dem Wege der Hydroformylierung von Olefinen erhältlich sind. Insbesondere sei darauf hingewiesen, daß beispielsweise auch substituierte Olefine, die im allgemeinen 1 bis 2, vorzugsweise einen Substituenten, tragen können, nach dem erfindungsgemäßen Verfahren hydroformyliert werden können. Beispielsweise können nach dem erfindungsgemäßen Verfahren ungesättigte, aliphatische Carbonsäureester, Acetale, Alkohole, Ether, Aldehyde, Ketone und Amine und Amide hydroformyliert werden. Als derartige substituierte Ausgangsolefine sind z.B. Methacrylsäureester, Dicyclopentadien, Vinyl- und Allylether, insbesondere entsprechende Derivate ungesättigter Fettsäuren von Interesse, beispielsweise die Ester der Öl-, Linol-, Linolen-, Ricinol- oder Erucasäure. Die aus diesen olefinischen Rohstoffen durch Hydroformylierung erhältlichen Aldehyde sind ebenfalls Ausgangsmaterialien zur Herstellung biologisch leicht abbaubarer, waschaktiver Substanzen.

Eine weitere Ausgestaltung der vorliegenden Erfindung betrifft Verfahren zur Herstellung verzweigter Carbonsäuren, Alkohole oder Amine aus α-Olefinen, wobei die α-Olefine nach dem erfindungsgemäßen Verfahren in der Vorcarbonylierungsstufe zu internen Olefinen isomerisiert, anschließend zu Isoaldehyden hydroformyliert und die so erhaltenen Isoaldehyde auf an sich herkömmliche Weise zu verzweigten Carbonsäuren oxidiert, zu verzweigten Alkoholen reduziert oder verzweigten Aminen reduktiv aminiert werden.

Die Oxidation der erfindungsgemäß aus α-Olefinen erhaltenen Isoaldehyde oder Isoaldehyd/n-Aldehyd-Gemische kann auf an sich herkommliche Weise, beispielsweise durch die Oxidation der Aldehyde mit Luftsauerstoff oder Sauerstoff gemäß den Verfahren, wie sie z.B. in Ullmann's Encyclopedia of Industrial Chemistry, 5. Aufl., Vol. A5, S.239, VCH Verlagsgesellschaft, Weinheim, 1986 dargestellt sind, erfolgen.

Die katalytische Hydrierung der nach dem erfindungsgemäßen Verfahren aus α-Olefinen erhältlichen Isoaldehyde oder Isoaldehyd/n-Aldehydgemische zu verzweigten Alkoholen kann auf an sich bekannte Weise, beispielsweise nach den Verfahren von Ullmann's Encyclopedia of Industrial Chemistry, 5. Aufl., Vol. A1, S.279, VCH Verlagsgesellschaft, Weinheim, 1985 oder G. H. Ludwig, Hydrocarbon Processing, März 1993, S.67, bewerkstelligt werden.

Die reduktive Aminierung der nach dem erfindungsgemäßen Verfahren aus α-Olefinen erhältlichen Isoaldehyde oder Isoaldehyd-/n-Aldehydgemische kann auf an sich bekannte Weise, beispielsweise nach den Verfahren von Ullmann's Encyclopedia of Industrial Chemistry, 5. Aufl., Vol. A2, S.1, VCH Verlagsgesellschaft, Weinheim, 1985 erfolgen. Als Ausgangsmaterial zur Herstellung von Aminen können sowohl Ammoniak, primäre C₁- bis C₂₀-Amine oder sekundäre C₂- bis C₂₀-Amine eingesetzt werden.

### Beispiel

Der Austrag aus der Hydroformylierung von Octen-N - einem Isomerengemisch aus Butendimeren - wurde mit einer 2 gew. -%igen Lösung von Tris-(natrium-m-sulfonatophenyl)phosphan (TPPTS) in Wasser extrahiert. Dabei wurde die wäßrige Lösung mit 80 Gew.-ppm Rhodium angereichert. 75 ml dieser Lösung wurden mit Octen-N im Autoklaven bei einem Kohlenmonoxid-Druck von 280 bar und bei einer Temperatur von 130°C 3 Stunden lang gerührt. Dabei wurde das an den Komplexbildner TPPTS gebundene Rhodium carbonyliert und aus dem wasserlöslichen Komplexbildner freigesetzt. Unter Druck wurden dem Autoklaven Proben der wäßrigen und organischen Phasen entnommen. Die organische Phase enthielt 84 Gew. -ppm, die wäßrige Phase 3 Gew. -ppm Rhodium. Der TPPTS-Komplexbildner war in der wäßrigen Phase verblieben.

Der Austrag aus dem Vorcarbonylierungsreaktor wurde unter Druck in eine wäßrige und eine organische Phase getrennt. Die organische Phase wurde anschließend in einem Hydroformylierungsreaktor bei 130°C und einem Synthesegasdruck (CO/H₂-Verhältnis: 1/1) von 280 bar hydroformyliert. Der Umsatz bei der Hydroformylierung betrug 93 %.

## Patentansprüche

1. Verfahren zur Herstellung von Aldehyden oder Aldehyden und Alkoholen durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe, dadurch gekennzeichnet, daß man den Rhodium-Katalysator aus dem Hydroformylierungsaustrag mittels einer wäßrigen Lösung eines wasserlöslichen, phosphorhaltigen Komplexbildners ausgewählt aus der Gruppe der einfach oder mehrfach sulfonierten Monound Oligophosphane, der einfach oder mehrfach sulfonierten Oligophosphite und/oder aus der Gruppe der einfach oder mehrfach carboxylierten Mono- oder Oligophosphane, in die wäßrige Phase extrahiert und aus dem extrahierten Hydroformylierungsaustrag den Aldehyd oder den Aldehyd und den Alkohol isoliert, den wäßrigen rhodiumhaltigen Extrakt einer Vorcarbonylierungsstufe zuführt und ihn in der Vorcarbonylierungsstufe in Gegenwart einer im wesentlichen wasserunlöslichen, organischen Flüssigkeit und in Gegenwart von Kohlenmonoxid oder eines Kohlenmonoxid-haltigen Gasgemischs bei einem Druck von 5 bis 600 bar und bei einer Temperatur von 50 bis 150°C einer Vorcarbonylierung unterzieht, den Austrag aus der Vorcarbonylierungsstufe in eine den Hauptteil des Rhodiums enthaltende organische Phase und in eine den Komplexbildner enthaltende wäßrige Phase trennt und die organische Phase zur Hydroformylierung des Olefins in Gegenwart von Synthesegas bei 50 bis 1000 bar und bei einer Temperatur von 60 bis 180°C der Hydroformylierungsstufe zuführt.

2. Verfahren nach Anspruch 1, dadurch gekennzeichnet, daß man die Extraktion des Rhodium-Katalysators aus dem Hydroformylierungsaustrag bei Temperaturen von 80 bis 140°C und bei einem Druck von 1 bis 20 bar durchführt.

3. Verfahren nach den Ansprüchen 1 und 2, dadurch gekennzeichnet, daß man die Extraktion des Rhodium-Katalysators einoder mehrstufig durchführt und die wäßrige Lösung des Komplexbildners bei der Extraktion bezüglich des Hydroformylierungsaustrags im Gegenstrom führt.

4. Verfahren nach den Ansprüchen 1 bis 3, dadurch gekennzeichnet, daß man die nach Abtrennung der den Hauptteil des Rhodiums enthaltenden organischen Phase nach der Vorcarbonylierungsstufe erhaltene, den Komplexbildner enthaltende wäßrige Phase zur Extraktion des Rhodium-Katalysators aus dem Hydroformylierungsaustrag verwendet.

5. Verfahren nach den Ansprüchen 1 bis 4, dadurch gekennzeichnet, daß das Verfahren in einer Anlage aus einer Vorcarbonylierungseinheit, einer Hydroformylierungseinheit und einer ein- oder mehrstufig arbeitenden Extraktionseinheit kontinuierlich durchgeführt wird.

6. Verfahren nach den Ansprüchen 1 bis 5, dadurch gekennzeichnet, daß man als Olefine unsubstituierte oder mit 1 oder 2 unter den Reaktionsbedingungen inerten Substituenten substituierte, lineare oder verzweigte α-Olefine oder interne Olefine oder Gemische dieser Olefine in An- oder Abwesenheit eines Lösungsmittels hydroformyliert.

7. Verfahren nach den Ansprüchen 1 bis 6, dadurch gekennzeichnet, daß man Propen- oder Buten-Oligomere oder -Cooligomere hydroformyliert.

8. Verfahren nach den Ansprüchen 1 bis 6, dadurch gekennzeichnet, daß man niedermolekulare Polyisobutene, Polybutadiene oder Isobuten-1,3-Butadien-Copolymere hydroformyliert.

9. Verfahren nach den Ansprüchen 1 bis 6, dadurch gekennzeichnet, daß man ungesättigte Fettsäurederivate hydroformyliert.

10. Verfahren nach den Ansprüchen 1 bis 9, dadurch gekennzeichnet, daß man
- den Austrag aus der Hydroformylierungsstufe in einer Extraktionsstufe A mit der wäßrigen Lösung des phosphorhaltigen Komplexbildners aus Extraktionsstufe C extrahiert,
- dieses Extraktionsgemisch in eine erste wäßrige und eine erste organische Phase trennt,
- die erste wäßrige Phase der Vorcarbonylierungsstufe und die erste organische Phase der Extraktionsstufe C zuführt,
- in der Vorcarbonylierungsstufe das in der ersten wäßrigen Phase enthaltene komplexierte Rhodium in Gegenwart einer im wesentlichen wasserunlöslichen, organischen Flüssigkeit mit Kohlenmonoxid, Synthesegas oder einem Kohlenmonoxid-enthaltenden Gasgemisch carbonyliert,
- den Austrag aus der Vorcarbonylierungsstufe in einer Phasenabscheidung B in eine zweite organische und eine zweite wäßrige Phase trennt,
- die zweite, das carbonylierte Rhodium enthaltende, organische Phase dem Hydroformylierungsreaktor und die zweite, den Komplexbildner enthaltende, wäßrige Phase der Extraktionsstufe C zuführt,
- in der Hydroformylierungsstufe das Olefin in Gegenwart von Synthesegas hydroformyliert,
- die zweite wäßrige Phase zur Extraktion von restlichem Rhodium-Katalysator aus der ersten organischen Phase in Extraktionsstufe C verwendet,
- das Extraktionsgemisch aus Extraktionsstufe C in eine dritte organische Phase und eine dritte wäßrige Phase trennt,
- aus der dritten organischen Phase den Aldehyd und/oder den Alkohol isoliert,
- und die dritte wäßrige Phase zur Extraktion des Rhodium-Katalysators aus dem Hydroformylierungsaustrag in die Extraktionsstufe A zurückführt.

11. Verfahren nach den Ansprüchen 1 bis 10, dadurch gekennzeichnet, daß man in der Vorcarbonylierungsstufe als im wesentlichen wasserunlösliche, organische Flüssigkeit einen Teil des Rohaustrags aus der Hydroformylierungsstufe verwendet.

12. Verfahren nach den Ansprüchen 1 bis 10, dadurch gekennzeichnet, daß man in der Vorcarbonylierungsstufe als im wesentlichen wasserunlösliche, organische Flüssigkeit einen Aldehyd oder Alkohol verwendet.

13. Verfahren nach Anspruch 12, dadurch gekennzeichnet, daß man den in der Hydroformylierungsstufe gebildeten Aldehyd oder Alkohol nach dessen Reinigung oder ein gereinigtes Gemisch aus diesem Aldehyd und Alkohol verwendet.

14. Verfahren nach den Ansprüchen 1 bis 10, dadurch gekennzeichnet, daß man in der Vorcarbonylierungsstufe als im wesentlichen wasserunlösliche, organische Flüssigkeit, Gemische hochsiedender Kondensationsprodukte von Aldehyden verwendet.

15. Verfahren nach den Ansprüchen 1 bis 10, dadurch gekennzeichnet, daß man in der Vorcarbonylierungsstufe als im wesentlichen wasserunlösliche, organische Flüssigkeit ein Olefin verwendet.

16. Verfahren nach den Ansprüchen 1 bis 10, dadurch gekennzeichnet, daß man in der Vorcarbonylierungsstufe als im wesentlichen wasserunlösliche Flüssigkeit das zu hydroformylierende α-Olefin einsetzt und die Vorcarbonylierung bei einem Druck von mehr als 100 bar und bei einer Temperatur von unter 110°C durchführt.

17. Verfahren nach den Ansprüchen 1 bis 10 und 15, dadurch gekennzeichnet, daß man in der Vorcarbonylierungsstufe als im wesentlichen wasserunlösliche organische Flüssigkeit ein α-Olefin einsetzt und die Vorcarbonylierung bei einem Druck von weniger als 100 bar und bei einer Temperatur von über 110°C durchführt.

18. Verfahren nach den Ansprüchen 1 bis 17, dadurch gekennzeichnet, daß man als phosphorhaltigen Komplexbildner ein wasserlösliches Salz des einfach, zweifach oder dreifach sulfonierten Triphenylphosphans verwendet.

19. Verfahren nach den Ansprüchen 1 bis 17, dadurch gekennzeichnet, daß man als phosphorhaltigen Komplexbildner ein wasserlösliches Salz des Tris(m-sulfonatophenyl)phosphans verwendet.

20. Verfahren nach den Ansprüchen 1 bis 17, dadurch gekennzeichnet, daß man als phosphorhaltigen Komplexbildner ein Sulfonat-Gruppen tragendes wasserlösliches Salz des 2,2'-Bis(diphenylphosphinomethyl)-1,1'-biphenyl verwendet.

21. Verfahren zur Herstellung von Carbonsäuren aus einem internen Olefin oder einem α-Olefin, dadurch gekennzeichnet, daß man das Olefin gemäß den Ansprüchen 1 bis 20 hydroformyliert und den dabei gebildeten Aldehyd nachfolgend in an sich bekannter Weise zur Carbonsäure oxidiert.

22. Verfahren zur Herstellung von Alkoholen aus einem internen Olefin oder einem α-Olefin, dadurch gekennzeichnet, daß man das Olefin gemäß den Ansprüchen 1 bis 20 hydroformyliert und den dabei gebildeten Aldehyd nachfolgend in an sich bekannter Weise zum Alkohol reduziert oder hydriert.

23. Verfahren zur Herstellung von Aminen aus einem internen Olefin oder einem α-Olefin, dadurch gekennzeichnet, daß man das Olefin gemäß den Ansprüchen 1 bis 20 hydroformyliert und den dabei gebildeten Aldehyd oder Alkohol in Gegenwart eines Aminierungskatalysators und Wasserstoff mit Ammoniak, einem primären oder sekundären Amin auf an sich bekannte Weise aminiert.

## Claims

1. A process for the preparation of aldehydes or aldehydes and alcohols by the hydroformylation of olefins of more than 3 carbon atoms, comprising the stage of hydroformylation by means of a rhodium catalyst homogeneously dissolved in the reaction medium, the separation of the rhodium catalyst from the discharge of the hydroformylation reaction and the recycling of the rhodium separated from the hydroformylation discharge into the hydroformylation stage, wherein the rhodium catalyst is extracted from the hydroformylation discharge into the aqueous phase by means of an aqueous solution of a water-soluble, phosphorus-containing complexing agent selected from the group consisting of the mono- or polysulfonated mono- and oligophosphines or the mono- or polysulfonated oligophosphites and/or from the group consisting of the mono or polycarboxylated mono or oligophosphines, and the aldehyde or the aldehyde and the alcohol is or are isolated from the extracted hydroformylation discharge, the aqueous rhodium-containing extract is fed to a precarbonylation stage and is subjected to a precarbonylation in the precarbonylation stage in the presence of an essentially water-insoluble organic liquid and in the presence of carbon monoxide or of a carbon monoxide-containing gas mixture at from 5 to 600 bar and from 50 to 180°C, the discharge from the precarbonylation stage is separated into an organic phase containing the main part of the rhodium and into an aqueous phase containing the complexing agent, and the organic phase is fed to the hydroformylation stage for hydroformylation of the olefin in the presence of synthesis gas at from 50 to 1,000 bar and at from 60 to 180°C.

2. A process as claimed in claim 1, wherein the extraction of the rhodium catalyst from the hydroformylation discharge is carried out at from 80 to 140°C and at from 1 to 20 bar.

3. A process as claimed in claims 1 and 2, wherein the extraction of the rhodium catalyst is carried out in one or more stages and the aqueous solution of the complexing agent is fed countercurrent to the hydroformylation discharge in the extraction.

4. A process as claimed in any of claims 1 to 3, wherein the aqueous phase obtained after removal of the organic phase containing the main part of the rhodium after the precarbonylation stage and containing the complexing agent is used for extracting the rhodium catalyst from the hydroformylation discharge.

5. A process as claimed in any of claims 1 to 4, wherein the process is carried out continuously in a plant comprising a precarbonylation unit, a hydroformylation unit and a onestage or multistage extraction unit.

6. A process as claimed in any of claims 1 to 5, wherein linear or branched α-olefins or internal olefins which are unsubstituted or substituted by 1 or 2 substituents which are inert under the reaction conditions, or mixtures of these olefins, are hydroformylated in the presence or absence of a solvent.

7. A process as claimed in any of claims 1 to 6, wherein propene or butene oligomers or cooligomers are hydroformylated.

8. A process as claimed in any of claims 1 to 6, wherein low molecular weight polyisobutenes, polybutadienes or isobutene/ 1,3-butadiene copolymers are hydroformylated.

9. A process as claimed in any of claims 1 to 6, wherein unsaturated fatty acid derivatives are hydroformylated.

10. A process as claimed in any of claims 1 to 9, wherein
- the discharge from the hydroformylation stage is extracted in an extraction stage A with the aqueous solution of the phosphorus-containing complexing agent from extraction stage C,
- this extraction mixture is separated into a first aqueous phase and a first organic phase,
- the first aqueous phase is fed to the precarbonylation stage and the first organic phase is fed to extraction stage C,
- in the precarbonylation stage, the complexed rhodium contained in the first aqueous phase is carbonylated in this carbon monoxide, synthesis gas or a carbon monoxide-containing gas mixture in the presence of an essentially water-insoluble, organic liquid,
- the discharge from the precarbonylation stage is separated in a phase separation B into a second organic phase and a second aqueous phase,
- the second organic phase containing the carbonylated rhodium is fed to the hydroformylation reactor and the second aqueous phase containing the complexing agent is fed to extraction stage C,
- in the hydroformylation stage, the olefin is hydroformylated in the presence of synthesis gas,
- the second aqueous phase is used for extracting residual rhodium catalyst from the first organic phase in extraction stage C,
- the extraction mixture from extraction stage C is separated into a third organic phase and a third aqueous phase,
- the aldehyde or the alcohol is isolated from the third organic phase,
- and the third aqueous phase is recycled to extraction stage A for extracting the rhodium catalyst from the hydroformylation discharge.

11. A process as claimed in any of claims 1 to 10, wherein some of the crude discharge from the hydroformylation stage is used in the precarbonylation stage as essentially water-insoluble, organic liquid.

12. A process as claimed in any of claims 1 to 10, wherein an aldehyde or alcohol is used in the precarbonylation stage as essentially water-insoluble, organic liquid.

13. A process as claimed in claim 12, wherein the aldehyde or alcohol formed in the hydroformylation stage is used after purification thereof or a purified mixture of this aldehyde and alcohol is used.

14. A process as claimed in any of claims 1 to 10, wherein a mixture of high-boiling condensates of aldehydes is used in the precarbonylation stage as essentially water-insoluble, organic liquid.

15. A process as claimed in any of claims 1 to 10, wherein an olefin is used in the precarbonylation stage as essentially water-insoluble, organic liquid.

16. A process as claimed in any of claims 1 to 10, wherein the α-olefin to be hydroformylated is used in the precarbonylation stage as essentially water-insoluble liquid and the precarbonylation is carried out at more than 100 bar and at below 110°C.

17. A process as claimed in any of claims 1 to 10 and 15, wherein an α-olefin is used in the precarbonylation stage as essentially water-insoluble organic liquid and the precarbonylation is carried out at less than 100 bar and at above 110°C.

18. A process as claimed in any of claims 1 to 17, wherein a water-insoluble salt of mono-, di- or trisulfonated triphenylphosphine is used as the phosphorus-containing complexing agent.

19. A process as claimed in any of claims 1 to 17, wherein a water-soluble salt of tris(m-sulfonatophenyl)phosphine is used as the phosphorus-containing complexing agent.

20. A process as claimed in any of claims 1 to 17, wherein a sulfo-carrying water-soluble salt of 2,2'-bis(diphenylphosphinomethyl)-1,1'-biphenyl is used as phosphorus-containing complexing agent.

21. A process for the preparation of carboxylic acids from an internal olefin or an α-olefin, wherein the olefin is hydroformylated as claimed in any of claims 1 to 20 and the aldehyde formed is subsequently oxidized to the carboxylic acid in a manner known per se.

22. A process for the preparation of alcohols from an internal olefin or an α-olefin, wherein the olefin is hydroformylated as claimed in any of claims 1 to 20 and the aldehyde formed is subsequently reduced or hydrogenated to the alcohol in a manner known per se.

23. A process for the preparation of amines from an internal olefin or an α-olefin, wherein the olefin is hydroformylated as claimed in any of claims 1 to 20 and the aldehyde or alcohol formed is aminated with ammonia or a primary or secondary amine in the presence of an amination catalyst and hydrogen in a manner known per se.

## Revendications

1. Procédé pour la préparation d'aldéhydes ou d'aldéhydes et d'alcools par l'hydroformylation d'oléfines ayant plus de 3 atomes de carbone, comprenant l'étape de l'hydroformylation au moyen d'un catalyseur à base de rhodium, dissous de façon homogène dans le milieu réactionnel, la séparation du catalyseur à base de rhodium d'avec le produit de la réaction d'hydroformylation et le recyclage, dans l'étape d'hydroformylation, du rhodium séparé du produit d'hydroformylation, caractérisé en ce que l'on extrait dans la phase aqueuse le catalyseur à base de rhodium, à partir du produit d'hydroformylation, au moyen d'une solution aqueuse d'un complexant phosphoré hydrosoluble, choisi dans le groupe des mono- et oligophosphanes mono- ou polysulfonés, des oligophosphites mono- ou polysulfonés et/ou dans le groupe des mono- ou oligophosphanes mono- ou polycarboxylés, et, à partir du produit d'hydroformylation extrait, on isole l'aldéhyde ou l'aldéhyde et l'alcool, on envoie l'extrait aqueux, contenant du rhodium, à une étape de précarbonylation, et on le soumet, dans l'étape de précarbonylation, à une précarbonylation sous une pression de 5 à 600 bar et à une température de 50 à 150°C, en présence d'un liquide organique pratiquement insoluble dans l'eau et en présence de monoxyde de carbone ou d'un mélange de gaz contenant du monoxyde de carbone, on scinde le produit, provenant de l'étape de précarbonylation, en une phase organique contenant la majeure partie du rhodium et en une phase aqueuse contenant le complexant, et on envoie la phase organique à l'étape d'hydroformylation, pour l'hydroformylation de l'oléfine en présence de gaz de synthèse, sous 50 à 1 000 bar et à une température de 60 à 180°C.

2. Procédé selon la revendication 1, caractérisé en ce que l'on effectue l'extraction du catalyseur à base de rhodium, à partir du produit d'hydroformylation, à des températures de 80 à 140°C et sous une pression de 1 à 20 bar .

3. Procédé selon les revendications 1 et 2, caractérisé en ce que l'on effectue l'extraction du catalyseur à base de rhodium en une ou plusieurs étapes, et, lors de l'extraction, la phase aqueuse du complexant est conduite à contre-courant par rapport au produit d'hydroformylation.

4. Procédé selon les revendications 1 à 3, caractérisé en ce que, dans l'extraction du catalyseur à base de rhodium à partir du produit d'hydroformylation, on utilise la phase aqueuse contenant le complexant, obtenue après l'étape de précarbonylation, après séparation de la phase organique contenant la majeure partie du rhodium.

5. Procédé selon les revendications 1 à 4, caractérisé en ce que le procédé est mis en oeuvre en continu dans une installation composée d'une unité de précarbonylation, d'une unité d'hydroformylation et d'une unité d'extraction fonctionnant en une ou plusieurs étapes.

6. Procédé selon les revendications 1 à 5, caractérisé en ce que l'on soumet à une hydroformylation, en tant qu'oléfines, des α-oléfines ou des oléfines internes, linéaires ou ramifiées, non substituées ou substituées par un ou deux substituants inertes dans les conditions réactionnelles, ou des mélanges de ces oléfines, en absence ou en présence d'un solvant.

7. Procédé selon les revendications 1 à 6, caractérisé en ce que l'on soumet à une hydroformylation des oligomères ou co-oligomères de propène ou de butène.

8. Procédé selon les revendications 1 à 6, caractérisé en ce que l'on soumet à une hydroformylation des polyisobutènes, polybutadiènes ou copolymères isobutène/1,3-butadiène à faible masse moléculaire.

9. Procédé selon les revendications 1 à 6, caractérisé en ce que l'on soumet à une hydroformylation des dérivés d'acides gras insaturés.

10. Procédé selon les revendications 1 à 9, caractérisé en ce que
- on extrait le produit provenant de l'étape d'hydroformylation, dans une étape d'extraction A, avec la phase aqueuse du complexant phosphoré provenant de l'étape d'extraction C,
- on scinde ce mélange d'extraction en une première phase aqueuse et une première phase organique,
- on envoie la première phase aqueuse à l'étape de précarbonylation et la première phase organique à l'étape d'extraction C,
- dans l'étape de précarbonylation, on soumet à une carbonylation le rhodium complexé, contenu dans la première phase aqueuse, en présence d'un liquide organique pratiquement insoluble dans l'eau, avec du monoxyde de carbone, du gaz de synthèse ou un mélange de gaz contenant du monoxyde de carbone,
- on scinde le produit, provenant de l'étape de précarbonylation, dans une séparation de phases B, en une deuxième phase organique et une deuxième phase aqueuse,
- on envoie au réacteur d'hydroformylation la deuxième phase organique contenant du rhodium carbonylé, et on envoie à l'étape d'extraction C la deuxième phase aqueuse contenant le complexant,
- dans l'étape d'hydroformylation, l'oléfine est hydroformylée en présence de gaz de synthèse,
- on utilise dans l'étape d'extraction C la deuxième phase aqueuse pour l'extraction du catalyseur résiduel à base de rhodium, à partir de la première phase organique,
- le mélange d'extraction provenant de l'étape d'extraction C est scindé en une troisième phase organique et une troisième phase aqueuse,
- on isole l'aldéhyde et/ou l'alcool à partir de la troisième phase organique,
- et la troisième phase aqueuse est recyclée dans l'étape d'extraction A, pour l'extraction du catalyseur à base de rhodium à partir du produit d'hydroformylation.

11. Procédé selon les revendications 1 à 10, caractérisé en ce que l'on utilise dans l'étape de précarbonylation une partie du produit brut provenant de l'étape d'hydroformylation, sous forme d'un liquide organique pratiquement insoluble dans l'eau.

12. procédé selon les revendications 1 à 10, caractérisé en ce que, dans l'étape de précarbonylation, on utilise comme liquide organique pratiquement insoluble dans l'eau un aldéhyde ou un alcool.

13. Procédé selon la revendication 12, caractérisé en ce que l'on utilise l'aldéhyde ou l'alcool formé dans l'étape d'hydroformylation, après sa purification, ou un mélange purifié de cet aldéhyde et de cet alcool.

14. Procédé selon les revendications 1 à 10, caractérisé en ce que, dans l'étape de précarbonylation, on utilise comme liquide organique pratiquement insoluble dans l'eau des mélanges de produits de condensation, à haut point d'ébullition, d'aldéhydes.

15. Procédé selon les revendications 1 à 10, caractérisé en ce que, dans l'étape de précarbonylation, on utilise comme liquide organique pratiquement insoluble dans l'eau une oléfine.

16. Procédé selon les revendications 1 à 10, caractérisé en ce que, dans l'étape de précarbonylation, on utilise comme liquide pratiquement insoluble dans l'eau l'α-oléfine devant être soumise à une hydroformylation, et on effectue la précarbonylation sous une pression de plus de 100 bar et à une température inférieure à 110°C.

17. Procédé selon les revendications 1 à 10 et 15, caractérisé en ce que, dans l'étape de précarbonylation, on utilise une α-oléfine comme liquide organique pratiquement insoluble dans l'eau, et on effectue la précarbonylation sous une pression de moins de 100 bar et à une température de plus de 110°C.

18. Procédé selon les revendications 1 à 17, caractérisé en ce que l'on utilise comme complexant phosphoré un sel hydrosoluble du triphénylphosphane mono-, bi- ou trisulfoné.

19. Procédé selon les revendications 1 à 17, caractérisé en ce que l'on utilise comme complexant phosphoré un sel hydrosoluble du tris(m-sulfonatophényl)-phosphane.

20. Procédé selon les revendications 1 à 17, caractérisé en ce que l'on utilise comme complexant phosphoré un sel hydrosoluble, portant des groupes sulfonate, du 2,2'-bis (diphénylphosphinométhyl) -1,1'-biphényle.

21. Procédé pour la préparation d'acides carboxyliques à partir d'une oléfine interne ou d'une α-oléfine, caractérisé en ce que l'oléfine est hydroformylée selon les revendications 1 à 20 et l'aldéhyde ainsi formé est ensuite oxydé en l'acide carboxylique, d'une façon connue en soi.

22. Procédé pour la préparation d'alcools à partir d'une oléfine interne ou d'une α-oléfine, caractérisé en ce que l'oléfine est hydroformylée selon les revendications 1 à 20, et l'aldéhyde ainsi formé est ensuite réduit ou hydrogéné en l'alcool, d'une façon connue en soi.

23. Procédé pour la préparation d'amines à partir d'une oléfine interne ou d'une α-oléfine, caractérisé en ce que l'oléfine est hydroformylée selon les revendications 1 à 20, et l'aldéhyde ou l'alcool ainsi formé est aminé, d'une façon connue en soi, avec de l'ammoniac, une amine primaire ou secondaire, en présence d'un catalyseur d'amination et d'hydrogène.
